# EUROPEAN PATENT APPLICATION

(11) **EP 3 824 944 A1**
(43) Date of publication of application: **26.05.2021**
(21) Application number: 19837532.1
(22) Date of filing: 16.07.2019
(51) Int. Cl.: A61N 1/00, A61N 1/04, A61N 1/18, A61N 1/36

(54) **PLATE ELECTRODES**

(30) Priority: 16.07.2018 CO 18007478
(71) Applicant: Panacea Quantum Leap Technology LLC, Dallas, TX 75202 (US)
(72) Inventor: VELASCO VALCKE, Francisco Javier, Bogotá (CO)
(74) Representative: Bryn-Jacobsen, Caelia
(86) International application number: PCT/IB2019/056079
(87) International publication number: WO 2020/016785

(57) **Abstract**

The present disclosure relates to electrodes for tissue electrical stimulation and methods of tissue electrical stimulation and administration of substances to such tissue using the electrode. In particular, it shows an electrode comprising: a first conductive plate; and a second conductive plate surrounding the first conductive plate; where the first conductive plate and the second conductive plate are separated from each other.

A method to induce an electric current with a tissue electrical stimulation electrode, in which an activation signal is applied to a first conductive plate of the electrode to generate an electric field inducing a current in the tissue and applying an electric potential to a second conductive plate of the electrode to obtain an electric potential differential with respect to the first conductive plate; wherein the electric potential of the second conductive plate forces the current induced by the first conductive material plate to penetrate the surface of the tissue in contact with the conductive plates and prevents the induced current from flowing on the surface of the tissue.

In addition, a method for supplying an ionically charged substance with the electrode shown in this document, which comprises placing a substance on the tissue and wherein by applying an electric field with sufficient force and duration the tissue cell walls become temporarily permeable and allow the substance to pass through them without damaging the tissue cells.

## Description

### Field of the invention

The present disclosure relates to electrodes, particularly with plate electrodes and optionally with plate electrodes which are concentric rings, overlapping each other or one of the plates surrounding the other plate, in order to send electrical impulses to tissues.

### BACKGROUND

Since the invention of the first electrode and given the advance and development of new non-invasive methods, it is clear the interest and need to develop better electrodes allowing a better electrical stimulation of tissues, being as less invasive as possible.

For example, neuronal treatment by means of electrical stimulation, as a medical treatment, has shown positive results in treating diseases such as epilepsy. However, in order to stimulate this tissue, it is necessary to use invasive techniques in many cases, because tissues surrounding the neural tissue, such as skin and bones of the skull, represent a barrier to electrical stimulation of neural tissue. These invasive techniques, although effective, increase the post-surgical recovery time, which is why advances are currently being sought in the development of technology capable of stimulating, in a transcranial, non-invasive manner, the tissues surrounding the neuronal tissue and, in general, tissues surrounding a tissue of interest to be stimulated.

Moreover, the art discloses tissue electrical stimulation electrodes, such as those disclosed by scientific article PMC3606894 Epilepsy Behav. 2013 Apr; 27(1): 154-158 and by patents EP2493374 B1 and US6135953 A.

The scientific article PMC3606894 Epilepsy Behav. 2013 Apr; 27(1): 154-158, shows a three-pole concentric ring electrode as a non-invasive alternative to stimulate the brain by carrying electrical signals through the skull. The article's electrode works with two concentric rings, which may focus the stimulation signal under the skull without altering brain activity, specifically in memory formation, as tests show.

Patent EP2493374 B1 shows a concentric ring electrode, with construction parameters obeying specific ranges relating to the diameter of a central ring-shaped plate and concentric ring-shaped plates. The electrode has a central node surrounded by at least two concentric rings to allow focusing the stimulus signal to the tissues.

Moreover, other electrodes with a different number of poles have also been developed for signal stimulation and measurement. The electrodes shown in patent US6135953 A, have conductive plates and are arranged in different ways, in particular, in one of the invention embodiments, a first electrode is shown with an interstice in the middle, where a second smaller electrode fits in. The first electrode is used as a dispersing plate, which functions as a return electrode for the electric currents circulating through a patient's body during electrical surgery, this causes the electric current focused on the second electrode with the smaller contact surface, to be dispersed over the relatively larger surface of the dispersing plate, reducing the power per square centimeter.

However, although the art discloses electrodes with concentric rings, it does not refer to a concentric ring with an electric potential barrier, which prevents the electric current that is induced to the tissue from being dispersed on the surface of the tissue. Due to this reason, the electrodes known in the art do not manage to adequately stimulate the tissue, since they do not allow the tissue continuous stimulation, which leads to long periods of treatment and heating of the tissue that could cause discomfort to the organism to which the treatment is being applied, even at the risk of damaging the tissue. The prior art encourages keeping the concentric rings coupled with the conductive plate, which prevents the application of an electrical potential as an electrical potential barrier on the surface around the central conductive plate.

### BRIEF DESCRIPTION

This disclosure relates to tissue electrical stimulation electrodes.

In particular, electrodes are shown comprising: a first conductive plate; a second conductive plate surrounding the first conductive plate; where the first conductive plate and the second conductive plate are separated from each other.

A method for inducing an electric current in a tissue, by means of an electrode of tissue electrical stimulation, is also disclosed, the method includes the following steps: A) locating the electrode of tissue electrical stimulation, including: a first conductive plate; and a second conductive plate surrounding the first conductive plate; wherein the first conductive plate and the second conductive plate are separated between them, in contact with the tissue; B) applying an activation signal to the first conductive plate of the electrode to generate an electric field inducing a current in the tissue; and C) applying an electric potential to the second conductive plate of the electrode to obtain an electric potential differential with respect to the first conductive plate; wherein the electric potential of the second conductive plate forces the current induced by the first conductive plate to penetrate the surface of the tissue in contact with the conductive plates and prevents the induced current from flowing through the surface of the tissue.

Furthermore, a method of delivering an ionically charged substance through the electrode is disclosed herein, which involves the following steps: before step A) of the method above, a substance is placed on the tissue, and in step B) the electrode is placed on the substance prepared, wherein the electric field of step B) is strong enough and durable enough to cause the tissue cell walls to become temporarily permeable, in order to allow the substance to pass through them without damaging the tissue cells.

### BRIEF DESCRIPTION OF THE FIGURES

FIG. 1 illustrates one form of disclosure and a detailed section view thereof.
FIG. 2 illustrates a diagram of a disclosure embodiment, with a substance in contact with the first conductive plate.
FIG. 3 illustrates a diagram of a disclosure embodiment, comprising a layer of electrically conductive material and a layer of sponge.
FIG. 4a shows an electrode configuration in which both conductive plates are made of a conductive material and sponge combination.
FIG. 4b shows an electrode configuration in which the first conductive plate is made entirely of conductive material, while the second conductive plate is made of sponge.
FIG. 4c shows an electrode configuration in which the two conductive plates are made entirely of sponge.
FIG. 4d shows an electrode configuration in which the first conductive plate is made entirely of sponge, while the second conductive plate is made of conductive material.
FIG 4e shows an electrode configuration in which the first conductive plate is made entirely of conductive material, while the second conductive plate consists of a conductive layer and a sponge layer.
FIG. 4f shows an electrode configuration in which the first conductive plate is made of a combination of conductive material and sponge and the second conductive plate is made of conductive material.
FIG. 5 illustrates a diagram of a disclosure embodiment, with a sponge in the interstice between the conductive plates.
FIG. 6A illustrates an example of a disclosure embodiment in which the first conductive plate comprises two conductive plates.
FIG. 6B illustrates an example of a disclosure embodiment in which the first conductive plate comprises three conductive plates.
FIG. 7A illustrates an example of a disclosure embodiment in which the first conductive plate and the second conductive plate are overlapping each other in an isometric view.
FIG. 7B illustrates an example of a disclosure embodiment in which the first conductive plate and the second conductive plate are overlapping each other in a front view, perpendicular to the plane where the conductive plates are inscribed.
FIG. 7C illustrates an example of a disclosure embodiment in which the first conductive plate and the second conductive plate are overlapping each other and separated by a dielectric, in a left side view.
FIG. 7D illustrates an example of a disclosure embodiment in which the first conductive plate and the second conductive plate are overlapping each other and in contact, in a left side view.
FIG. 8 illustrates a flow chart of a method for inducing into a tissue an electric current with a tissue electrical stimulation electrode.
FIG. 9 illustrates a flow chart of a method for administering to a tissue a substance with a tissue electrical stimulation electrode.

### DETAILED DESCRIPTION

The present disclosure refers to electrodes and methods to electrically stimulate organic tissues, as well as methods to induce electric currents by means of electrodes in organic tissues and methods to administer substances to organic tissues, by means of electrodes.

In this disclosure, the organic tissue or tissue, refers to biological tissues of living beings consisting of one or more cells of a single class or several types of cells, which form an organ or organism. The tissue may be healthy tissue, such as epithelial tissue, connective tissue, muscle tissue, nerve tissue, among others. The tissue may also be a tissue with a total or partial biochemical imbalance, which in turn may correspond to benign neoplastic tissue, malignant neoplastic tissue or any cell outside of homeostasis. Also, the tissue may correspond to in vivo cells or prior to the implantation of such cells in an in vivo environment. Optionally, the tissue may refer to cells in an ex vivo environment, for example in vitro cells.

The tissue may come from animals, which include, without limitation: mammals, bird species (including chickens, turkeys, geese and ducks, among others), fish, crustacean species (shrimp, lobster, crayfish, among others) and reptiles (such as crocodiles and alligators, among others). The term "mammal", as used in this document, refers to any species classified as mammal, including humans, non-human primates, such as cynomolgus monkeys, chimpanzees, baboons, and gorillas; domestic and farm animals including equine species, bovine species, porcine species, goat species, canine species, feline species, sheep species, rabbits, llamas; ungulates, such as bovine, sheep, swine, equine, goat; canine, feline, murine, rabbit; and rodents such as guinea pigs, hamsters and rats; among others.

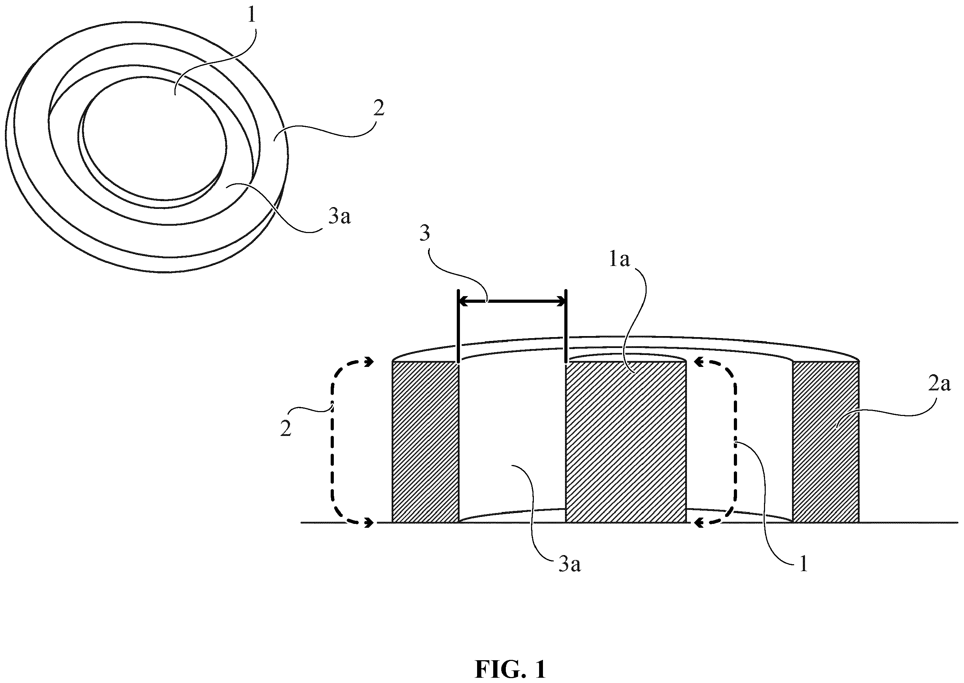

Referring to FIG. 1, an embodiment of the electrodes in the disclosure is illustrated, and a section view with a detail thereof, where the electrode comprises: a first conductive plate (1); and a second conductive plate (2) surrounding the first conductive plate (1); where the first conductive plate (1) and the second conductive plate (2) are separated from each other by an interstice (3).

In other embodiments, the interstice (3) is occupied by a gas (3a), which is selected from the group formed by air, industrial gases such as carbon dioxide acetylene (C₂H₂), carbon dioxide (CO₂), carbon monoxide (CO), chlorine (Clₓ), hydrogen (H₂) hydrogen chloride (HCl), methane (CH4), nitrous oxide (N₂O), propane (C₃H₈), sulfur dioxide (SO₂) and air gases such as argon (Ar), nitrogen (N₂), oxygen (O₂).

Optionally, the electrode has conductive plates (1) and (2) electrically isolated from each other, allowing the creation of an electric potential barrier to force an induced current into the tissue; the electric potential barrier will be described later. In another specific embodiment of the electrode, the conductive plates (1) and (2) are electrically connected to each other.

The electrode of this disclosure may be manufactured from a combination of materials, in which one part of the electrode is composed of sponge and the other part comprises solid conductive metals (1a and 2a). Thus, the first conductive plate (1) and the second conductive plate (2) are manufactured from conductive materials which are selected, without limitation, from the group consisting of, among others, zinc, magnesium, tin, aluminum, silver, platinum, copper, gold, aluminum, iron, lead, mercury, nicrom, nickel, chrome, carbon, indium, gallium, graphite and combinations thereof.

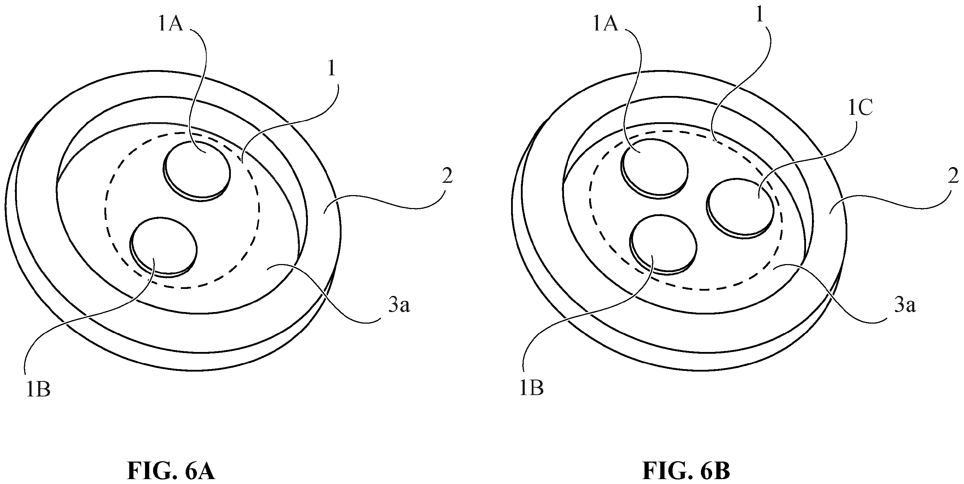

In particular electrode embodiments, the first conductive plate (1) may be an electrode array as shown in FIG. 6A and FIG. 6B.

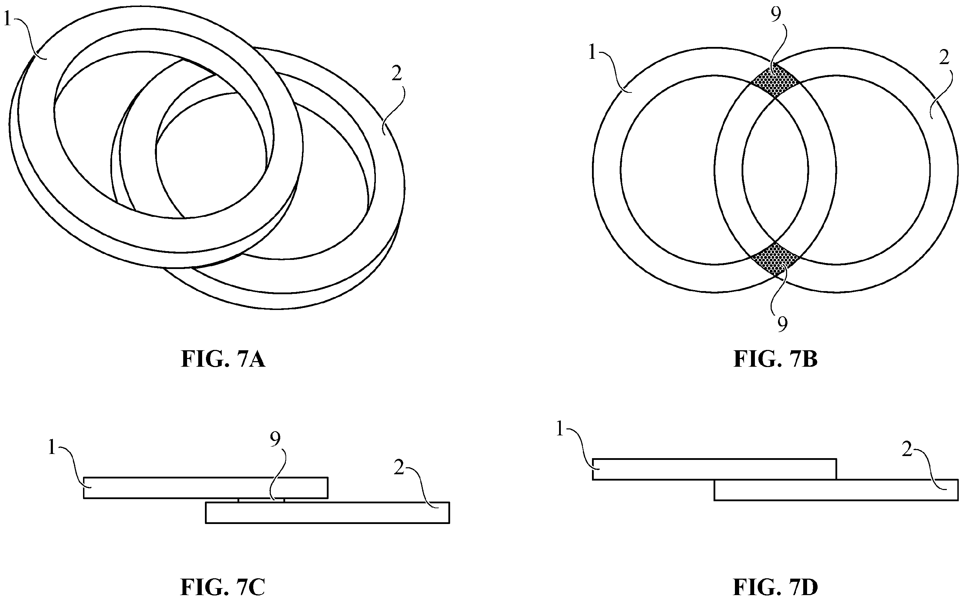

The plate electrode plates may be circular and be configured concentrically as in FIG. 1 through FIG. 5, or interior as shown in FIG. 6B and FIG. 6B, or be overlapping each other as shown in the figures from FIG. 7A through FIG. 7D. Also, in embodiments not illustrated, the conductive plates (1) and (2) of the electrode are secant to each other, i.e., they cut each other.

In another embodiment not illustrated, the conductive plates (1) and (2) that are secant to each other may also be electrically isolated despite being secant, so both conductive plates (1) and (2) when used to stimulate a tissue, may be in contact with the tissue using a method with an independent activation signal for each conductive plate (1 and 2), improving the efficiency of tissue stimulation compared to the embodiment illustrated in figures FIG. 7A to FIG. 7D.

However, the conductive plates (1) and (2) may have different geometric shapes as described below.

On the other hand, the dimensions of the electrode may be between 0.001 cm and 1 meter, without being limited to this range, as it varies depending on the dimensions of the tissue to be stimulated. Optionally, the dimensions of the electrode are between 0.001 cm and 40 cm.

In the particular embodiment, when the conductive plates (1) and (2) are overlapping each other, they may be electrically isolated from each other by means of a dielectric (9) as shown in FIG. 7B and FIG. 7C. Also, the conductive plates (1) and (2) may be electrically connected in the area where the conductive plates make contact.

Referring to FIG. 7B, an embodiment of the electrode is illustrated in a front view, perpendicular to the plane where the conductive plates (1) and (2) are inscribed. In the illustrated embodiment the conductive plates (1) and (2) are circular and are overlapping each other but separated by a dielectric (9).

Referring to FIG. 7C, the electrode embodiment illustrated in FIG. 7B is illustrated in a left side view.

Referring to FIG. 7C is illustrated in a left side view of an electrode embodiment variation illustrated in FIG. 7B in which the conductive plates (1) and (2) are in electrical contact with each other.

The different electrode shapes, dimensions and configurations allow for efficient tissue stimulation without stimulating regions of tissue that for practical reasons should not be stimulated, such as high sensitivity, or healthy regions of tissue that do not need stimulation. The different shapes of the electrode in conjunction with the method disclosed below, make it possible to control the currents that are induced in the tissue.

In an electrode embodiment of the present disclosure, the electrode comprises sponges, which may or may not be electrically conductive, where the first conductive plate (1) and/or the second conductive plate (2), are made of sponge. It should be noted that for the sponge to function as a conductor, a conductive liquid or gel (6) is required with which the sponge is moistened and allows electrical conduction, e.g., a liquid with a diluted electrolyte. Thus, optionally, the electrode made of sponge is moistened with conductive liquids or gels (6), so the liquid or gel with which the electrode is moistened contributes to establishing the electrical contact between the electrode and the tissue to be stimulated. The sponge may also store a substance that is administered to a tissue, this procedure will be detailed later.

In embodiments of the present disclosure, the first conductive plate (1) may be made of sponge, while the second conductive plate (2) is made of a conductive material. Other combinations are possible, and should be understood as parts of the same disclosure, e.g., it is possible that the ring-shaped conductive plate (2) is made of sponge, e.g., natural or synthetic sponge. It is also possible that both the first conductive plate (1) and the second conductive plate (2) are made of sponge.

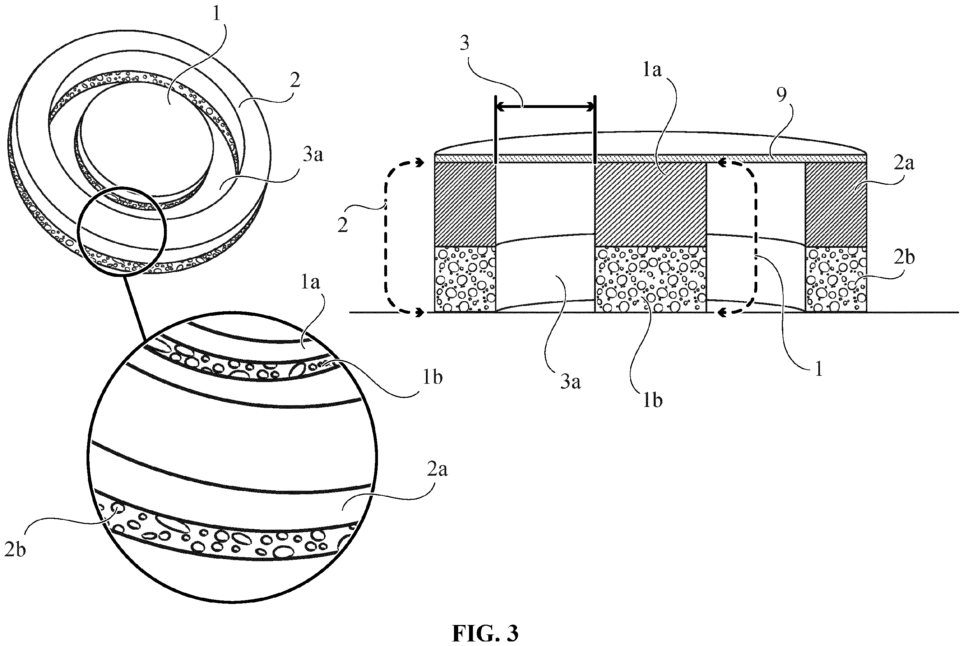

Referring to FIG. 3, in an example of the present disclosure, the body of the electrode comprises a layer of dielectric material (9), on which the first conductive plate (1) and the second conductive plate (2) are arranged, so they are fixed in position.

Optionally, the layer of dielectric material (9) serves as a support on which the electrode is built, and it is made of selected dielectrics, among others, from the group formed by glass, ceramic, rubber, mica, wax, paper, dry wood, porcelain, Bakelite, and combinations thereof.

This coating of dielectric material (9) also prevents oxidation of the conductive plate it covers (1 and 2). In addition, this coating of dielectric material (9) also allows the electrode to be electrically uncoupled from the tissue to be stimulated. Electrically uncoupling the electrode from the tissue to be stimulated is useful to protect the tissue from electrical discharges which could damage it.

For the understanding of the present disclosure, it will be understood that electrical uncoupling corresponds to disconnect two electrical circuits.

In another optional embodiment of the electrode, the conductive plates (1) and (2) are covered by a protective material that prevents the conductive plates (1) and (2) from degrading or oxidizing on contact with air or oxidizing gases.

The conductive plates (1) and (2) have a shape that is selected, among others, from the group formed by two-dimensional shapes such as rectangles, circles, ovals, parallelograms, rhombuses, etc. or three-dimensional forms such as spheres, cylinders, polyhedrons, prisms, revolution toroids, etc. or combinations thereof. The conductive plates (1) and (2) may have the same shape or different shapes between them.

Alternatively, the conductive plates (1) and (2) of the electrode are electrically isolated concentric rings, and operate with the same methods mentioned, where around the second conductive plate (2), at least one additional conductive plate is arranged around the second conductive plate (2), in a concentric manner, i.e., forming more than one ring around the first conductive plate (1). Optionally, the concentric rings are electrically connected to each other.

Alternatively, the first conductive plate (1) may be a commercially available electrode, which is arranged in the middle of an insulating material layer (9) that is used as a base, while the second conductive plate (2) is arranged, so it surrounds the first conductive plate (1) without touching each other.

In another optional embodiment of the electrode disclosed, between the first conductive plate and the second conductive plate (2), a dielectric material is provided (9). The dielectric material (9) allows a higher voltage to be reached between the conductive plates (1) and (2), so that a tissue located at a greater depth may be stimulated, such as the tissue of a kidney that is located at a certain depth under the skin tissue that covers an animal's abdominal area.

Referring to FIG. 6A, an example of a disclosure embodiment is illustrated, in which the first conductive plate (1) comprises two conductive plates (1A and IB).

Referring to FIG. 6B, an example of a disclosure embodiment is illustrated, in which the first conductive plate (1) comprises three conductive plates (1A, 1B and 1C).

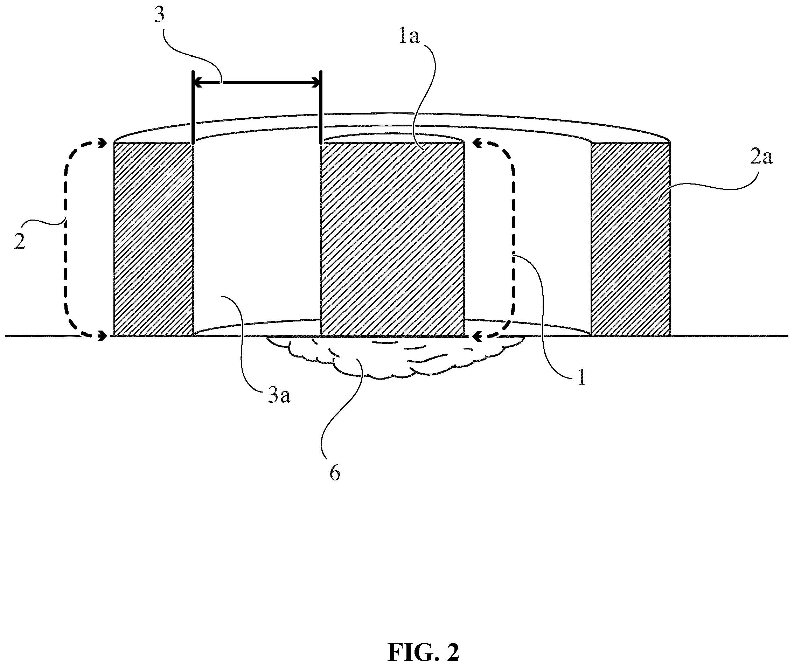

Referring to FIG. 2, in another form of disclosure, a substance (6), which is for example a gel or hydrogel, is used to bring the first conductive plate (1) or the second conductive plate (2), or both, into contact with the tissue, maintaining the electrical insulation between them.

An electrical potential barrier may also be applied through the second conductive plate (2), and the electrical signals are induced with the first conductive plate (1). The electric potential barrier corresponds to a region of the tissue with an electric field intensity formed by the electric potential applied by the second conductive plate (2), which opposes the passage of electrically charged particles, in a direction that depends on the electric charge signal.

On the other hand, it must be understood that in particular embodiments, for the use of the electrode a conductive gel or hydrogel (6) is used to make contact between the electrode and the tissue, this hydrogel reduces the resistance on the tissue surface.

The interstice (3) may be left free for air to serve as a dielectric (9) or use a known commercially available dielectric (9) to keep the plates separated from each other. The interstice (3) may be filled with dielectrics from the paper and *polyvinyl chloride* (PVC) group, amber, Bakelite, 7740 Pyrex glass, nylon, silicone, polyethylene, polystyrene, porcelain, rubber, neoprene, Teflon, titanium oxide, or combinations thereof, among others.

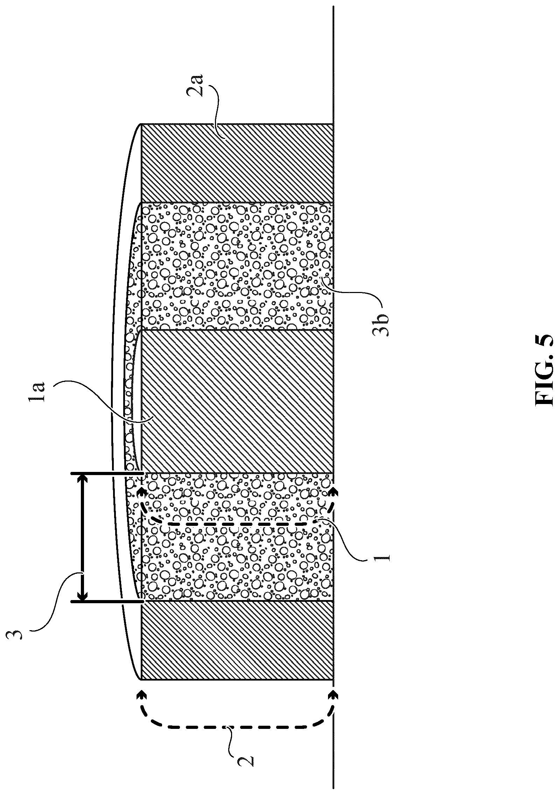

Referring to FIG. 5, a diagram of a spreading embodiment is illustrated with a sponge (3b) in the interstice (3) between the conductive plates (1) and (2).

The sponge (3b) serves as a dielectric (9) between the conductive plates (1) and (2), optionally it serves as a support for the same plates of the same electrode. One of the advantages of the sponge (3b) is that it may be used to absorb a substance that is subsequently administered to a tissue using a method of this disclosure.

Optionally in a specific embodiment, the sponge (3b) is moistened with a liquid desiccant from which an adsorbed substance is extracted using electrical fields commanded by an activation signal and an electrical potential supplied to the electrode of the disclosure, as will be described later, and the substance extracted from the liquid desiccant is then administered to the tissue. The substance administered to the tissue serves to increase the electrical conduction to the tissue.

The conductive plates (1) and (2) may be made of sponge, which is moistened with a gel or a conductive liquid (6), which may be a saline solution.

Optionally, the disclosed electrode may be operated with iontophoresis methods. Likewise, this document presents a stimulation method that makes use of concentric electrodes with which an improved method of tissue electrical stimulation is achieved.

The first conductive board (1) and the second conductive board (2) may be connected to devices that generate an activation signal, such as a magnetic function generator as described in application no. NC2017/000264, filed on November 17, 2017. Thus, the first conductive board (1) is connected, according to its application, to a device which is selected from the group consisting of a signal generator, a measuring device, a tissue stimulation device, electric massage devices, for example an analog-digital circuit of a data acquisition board or a controller, among others.

It is possible, for example, for the first carrier plate (1) and the second carrier plate (2) to be electrically connected to a signal generating device or directly to a computer unit, these plates may be isolated or electrically connected to each other, if the application requires it.

Referring to FIG. 3, in other embodiments, both the first conductive plate (1) and the second conductive plate (2), placed on the same layer of insulating material (9) separated by an air interstice (3a), also have a sponge layer (1b) of the first conductive plate (1) and a sponge layer (2b) of the second conductive plate (2).

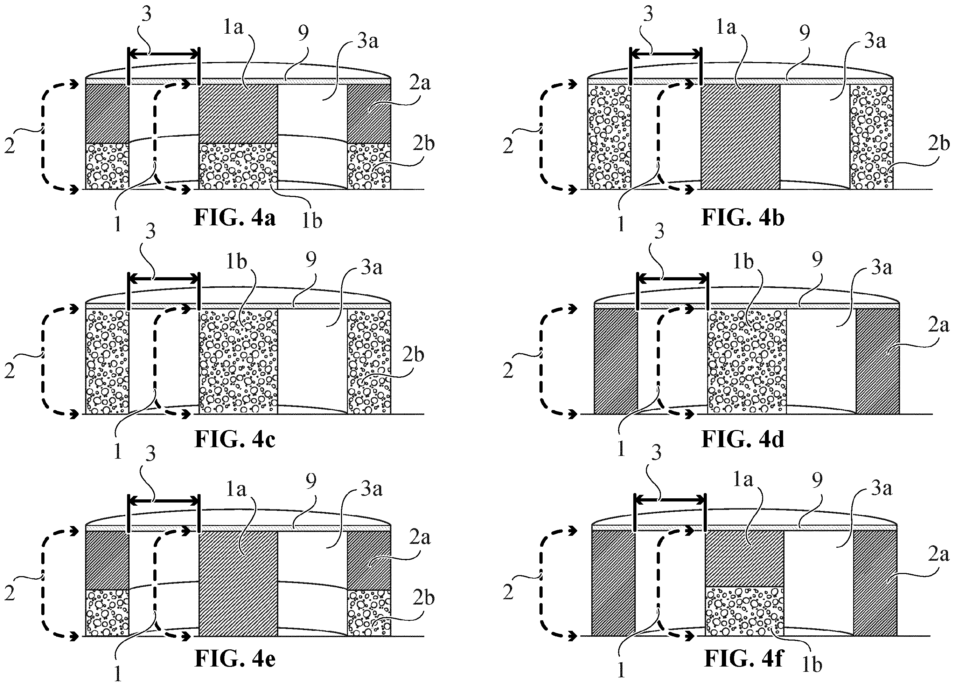

Referring to FIG. 4a through FIG. 4f, different configurations of conductive plates (1) and (2) and electrode sponges of the present disclosure are illustrated.

Referring to FIG. 4a, an electrode configuration is shown in which both conductive plates (1) and (2) are made of a combination of conductive material (1a and 2a) and sponge (1b and 2b).

Referring to FIG. 4b, an electrode configuration is shown in which the first conductive plate (1) is made entirely of conductive material (4b), while the second conductive plate (2) is made of sponge (2b).

Referring to FIG. 4c, an electrode configuration is shown in which the two conductive plates (1) and (2) are made entirely of sponge (1b and 2b).

Referring to FIG. 4d, an electrode configuration is shown in which the first conductive plate (1) is made entirely of sponge (1b), while the second conductive plate (2) is made of conductive material (2a).

Referring to FIG. 4e, an electrode configuration is shown in which the first conductive plate (1) is made entirely of conductive material (1a), while the second conductive plate (2) consists of a conductive layer (2a) and a sponge layer (2b).

Referring to FIG. 4f, an electrode configuration is shown in which the first conductive plate (1) is made of a combination of conductive material (1a) and sponge (1b) and the second conductive plate (2) is made of conductive material (2a).

On the other hand, the modalities illustrated in FIG. 6A, FIG. 6B, FIG. 7A, FIG. 7B, FIG. 7C, FIG. 7D, may also have the conductive plate (1) and (2) and sponge configurations illustrated in the figures from FIG. 1 to FIG. 5.

In these embodiments from FIG. 4a to FIG. 4f, the sponge (1b and 2b) is moistened with a conductive substance (6) that allows electrical conduction through the sponge (1b and 1b), and also reduces the electrical resistance on the surface of the tissue. In addition to the conductive material (1a and 2a).

Alternatively, the sponge (1b and 2b) can be moistened with an ionically charged substance to be administered by a technique, such as iontophoresis. Due to the shape of the electrode described herein, its function is not limited to the acquisition or administration of electrical signals, e.g., a method of administering medication to a tissue, using the iontophoresis technique, may be employed.

Thus, with the electrode disclosed, a substance may be conducted to pass through the surface of the tissue, through the processes of electrophoresis, electroosmosis, increase the permeability of the surface of the tissue with an electric field or a combination thereof.

The substance may be any medication.

Also, the electrode disclosed, in conjunction with the method of substance administration disclosed herein, are used to administer substances to a tissue, these substances may be strong electrolytes that when dissolved in water produce loose ions, creating an ionically charged solution which conducts electricity. The substances dissolved in water are selected from the group formed by salt, nitric acid, hydrochloric acid, sodium hydroxide, copper sulphate solution, sodium chloride, molten gold, molten aluminum and combinations thereof, among others.

For the iontophoresis method, a signal is applied on the first conductive plate (1) where it generates an electric field strength, in a range between 2V/cm and 5V/cm, and frequencies between 0.1 Hz and 50 MHz, preferably between 0.1 Hz and 500 kHz.

Optionally, in order for the substance to be administered ionically to the tissue, the electric field intensity must exceed the ionic charge of the substance.

Alternatively, in all embodiments using sponges, with a sponge (1b), a sponge (2b) or a sponge (3b), these sponges are replaced by a reservoir with a permeable membrane. This reservoir serves to accommodate solid gels or desiccants that have previously adsorbed an ionically charged substance.

Using the method of administering a substance it is possible to extract or regenerate the liquid that has been adsorbed by the solid desiccant to be administered to the tissue using electrical fields. According to the activation signal used to activate the conductive plates (1) and (2), the release of the ionic-charged substance from the desiccant is modulated and dosed into the tissue.

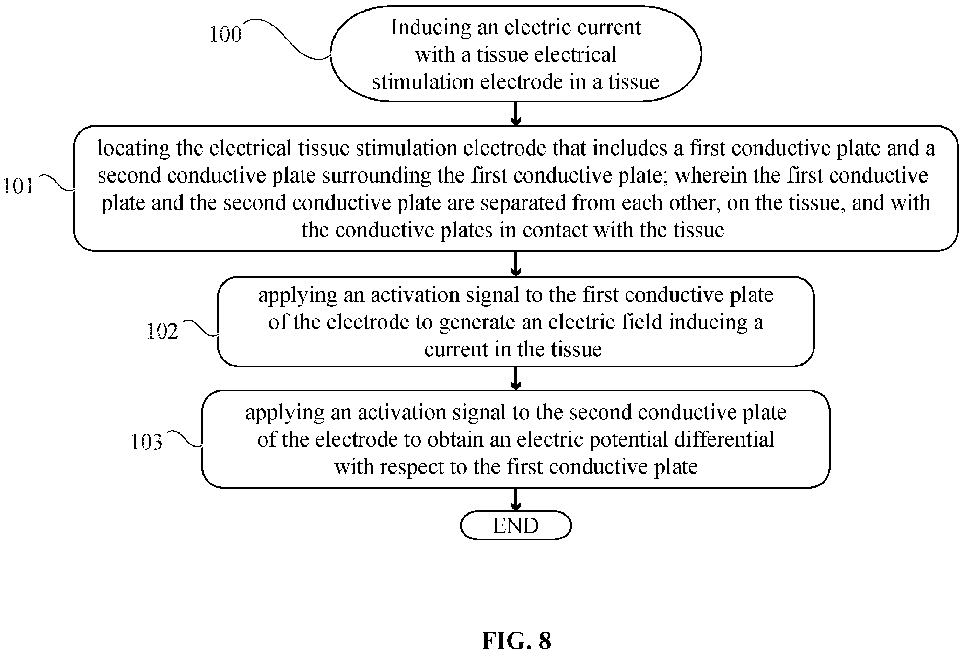

Referring to FIG. 8, a flow chart of a method to induce an electric current into a tissue, with a tissue electrical stimulation electrode, is illustrated, where the method (100) comprises the following steps: (101) A) locating the tissue electrical stimulation electrode including: a first conductive plate (1); and a second conductive plate (2) that surrounds the first conductive plate (1); where the first conductive plate (1) and the second conductive plate (2) are separated between them, on the tissue and with the conductive plates (1) and (2) in contact with the tissue; (102) B) applying an activation signal to the first conductive plate (1) of the electrode to generate an electric field inducing a current in the tissue; and (103) C) applying an electric potential to the second conductive plate (2) of the electrode to obtain an electric potential differential with respect to the first conductive plate, where the electric potential of the second conductive plate (2) forces the current induced by the first conductive plate (1) to penetrate the surface of the tissue in contact with the conductive plates (1) and (2) and prevents the induced current from flowing through the surface of the tissue. The way the electrode is constructed allows the stimulation of tissues underneath the tissue.

In an example, both in step B) and in step C), applying an activation signal and applying an electrical potential corresponds to providing a voltage to a terminal of electrical power supply of the conductive board (1 and/or 2), this voltage may be fixed or variable in time.

When the conductive plates (1) and (2) are in electrical contact, a single activation signal or electrical potential is required to create an electrical field that stimulates the tissue.

In embodiments where the conductive plates (1) and (2) are not electrically connected or are electrically isolated, several activation signals may be used for each conductive plate (1 and 2), allowing it to function as an electrical potential barrier.

The activation signals applied to the electrode on its conductive plates (1) and (2) may be out of phase or have different frequencies. For example, an electrode such as the one disclosed, on which an activation signal with a frequency of 2000 Hz and a voltage of 20 V is applied to the first conductive plate (1), while on the second conductive plate (2) an electrical potential with a frequency of 2020 Hz and a voltage of 24 V is applied.

Optionally, the electrical potential applied on the second conductive plate (2) is positive when the activation signal is positive, and negative when the activation signal is negative.

In an embodiment of the disclosed method, the activation signal is selected from the group consisting of alternating current signal or direct current signal, pulsed signal, alternating or non-alternating pulse train, square signal with useful cycle variation, triangle wave signal, sawtooth wave signal, Amplitude Modulation (AM), Frequency Modulation (FM), Phase Modulation (PM), Pulse Position Modulation (PPM) or combinations thereof.

Optionally, the electrode and the method of inducing an electric current with a tissue electrical stimulation electrode in a tissue, allows a medication to be administered below the surface of a tissue. For this purpose, a current flow up to a maximum of 1 mA (milliampere)/cm² (square centimeter) is used. Generally, current administration does not exceed a duration of 3 minutes, because it may cause burns and local irritation. A point for the application of medicines does not exceed the current density of 0.5 mA/cm², commonly working up to 0.4 mA/cm².

As another example, the signal applied to treat excessive sweating lasts between 20 to 40 minutes, daily and reduce the frequency of use (once a week) and usually takes 8 to 12 times until the expected reduction in sweating is achieved. The specific current for this type of treatment varies between 15 mA and 20 mA direct current.

In a further example of the present disclosure, the substance used is an ionically charged substance, its charge is either positive or negative. The activation signal used to administer such substance is of the same electrical nature, i.e., for an ionically negative substance a net negatively charged activation signal is used and if the substance is ionically positive, then the signal is net positively charged.

According to the previous example, in a particular embodiment of the method to induce an electric current with a tissue electrical stimulation electrode in a tissue, a method is configured to deliver an ionically charged substance with the electrical stimulation electrode, which comprises the following steps: before step A), a substance is placed on the tissue and in step B) the electrode is placed on the placed substance, where the electric field of step B) is strong enough and long enough to cause the tissue cell walls to become temporarily permeable to allow the substance to pass through them without damaging the tissue cells. Referring to FIG. 2, in another embodiment of the disclosure, the device is placed on the surface of the tissue to apply a substance (6), which may or may not be ionically charged. With the first conductive plate (1) a positive or negative electric field is generated, contrary to the charge of the substance (6), an electric potential barrier is generated on the tissue with the second conductive plate (2) charging it with an electric signal of reverse polarity to the signal on the first conductive plate (1).

In an alternative embodiment, the activation signal applied in step B) has a frequency between 0.1 Hz and 50 MHz and a voltage variation between 1 V and 24 V. Optionally, the activation signal applied in step B) has a frequency between 0.1 Hz and 500 kHz and a voltage variation between 1 V and 24 V.

It is possible that the electrode works at a voltage higher than 24 V according to the application in which it is used, this to maintain an electric field that manages to stimulate the tissue interior without damaging it.

In another particular embodiment of the disclosed method, the electric field of step B) has an intensity between 2V/cm and 5V/cm. In these ranges of electric field intensity, it is possible to stimulate the tissue with the electrode in contact with the tissue, without damaging it. Alternatively, the electrode in this configuration is an electrode covered with a dielectric material.

In one example of the method in which a substance is administered to the tissue with a tissue electrical stimulation electrode, the substance is injected into the tissue.

In another example of the method to induce an electric current with a tissue electrical stimulation electrode, more than one electrode is placed on different parts of a body.

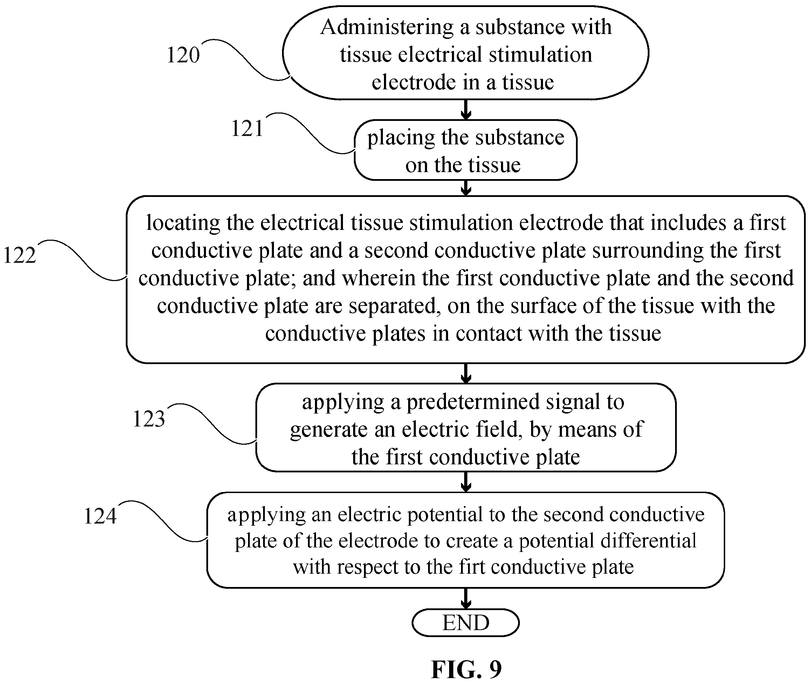

Referring to FIG. 9, a flow chart of a method for administering a substance with a tissue electrical stimulation electrode into a tissue is illustrated (120), the method (120) comprises the following steps: (121) a) disposing the substance on the tissue; (122) b) locating the tissue electrical stimulation electrode including: a first conductive plate; and a second conductive plate surrounding the first conductive plate; and where the first conductive plate and the second conductive plate are separated from each other, on the surface of the tissue with the conductive plates in contact with the tissue; (123) c) applying an activation signal to the first conductive plate of the electrode to generate an electric field, which induces a current in the tissue; and (124) d) applying an electrical potential to the second conductive plate of the electrode to obtain an electrical potential differential with respect to the first conductive plate; where the electrical field of the step (123) c) is strong enough and durable enough to cause the tissue cell walls to become temporarily permeable to allow the substance to enter them without damaging the tissue cells.

In an alternative embodiment, the activation signal applied in step (123) c) has a frequency between 0.1 Hz and 50 MHz and a voltage variation between 1 V and 24 V. Optionally, the activation signal applied in step (123) c) has a frequency between 0.1 Hz and 500 kHz and a voltage variation between 1 V and 24 V.

In a particular embodiment of the disclosed method, the electric field of the step (123) c) has an intensity between 2V/cm and 5V/cm. In these ranges of electric field intensity, it is possible to stimulate the tissue with the electrode in contact with the tissue, without damaging it. Alternatively, the electrode in this configuration is an electrode covered with a dielectric material

Alternatively, the substance is ionically charged, and where the electric field is equal to or greater than the charge of the substance.

In one example of the method in which a substance is administered to the tissue with a tissue electrical stimulation electrode, the substance is injected into the tissue.

In another example of the method of inducing an electric current with a tissue electrical stimulation electrode, more than one electrode is placed on different parts of a body. The activation signal to stimulate the tissue in contact with the first conductive plate (1) is in a range of voltages that allow generating an electric field intensity between 2V/cm and 5V/cm, and a frequency range between 0.1 Hz and 50 MHz, optionally, the frequency range is between 0.1 Hz and 500 kHz.

The present disclosure is not limited to the described and illustrated embodiments, because as it will be evident for a person versed in the art, there are possible variations and modifications that do not deviate from the disclosure spirit, which is only defined by the following claims.

## Claims

1. A tissue electrical stimulation electrode comprising:
- a first conductive plate; and
- a second conductive plate surrounding the first conductive plate;
where the first conductive plate and the second conductive plate are separated from each other.

2. The electrode of Claim 1, wherein the first conductive plate is made of sponge.

3. The electrode of Claim 1, wherein the second conductive plate is made of sponge.

4. The electrode of Claim 1, wherein the first conductive plate and the second conductive plate are made of sponge.

5. The Claim 1 electrode, wherein there is at least one additional conductive plate to the second conductive plate.

6. The electrode of Claim 1, wherein between the first conductive plate and the second conductive plate there is a dielectric material.

7. The electrode in Claim 1, wherein the first and second conductive plate consist of a conductive layer with a sponge layer.

8. The Claim 1 electrode, wherein the electrode body consists of a layer of insulating material that supports the first conductive plate and the second conductive plate.

9. The Claim 1 electrode, wherein the first conductive plate and the second conductive plate are coated with an insulating material.

10. The electrode of Claim 1, wherein around the second conductive plate there is at least one additional conductive plate, which surrounds the second conductive plate, in a concentric manner.

11. The Claim 1 electrode, for use in a tissue electrical stimulation machine.

12. The Claim 1 electrode wherein the conductive plates are made of selected conductive materials from the group comprising zinc, magnesium, tin, aluminum, silver, platinum, copper, gold, aluminum, iron, lead, mercury, nicrom, nickel, chrome, carbon, indium, gallium, graphite and combinations thereof.

13. A method of inducing an electric current with a tissue electrical stimulation electrode in a tissue, the method comprises the following steps:
A) locating the electrode of electrical stimulation of tissues, including:
- a first conductive plate; and
- a second conductive plate surrounding the first conductive plate; wherein the first conductive plate and the second conductive plate are separated from each other, on the tissue and with the conductive plates in contact with the tissue;
B) applying an activation signal to the first conductive plate of the electrode to generate an electric field that induces a current in the tissue; and
C) applying an electrical potential to the second electrode conductive plate to obtain an electrical potential differential with respect to the first conductive plate;
wherein the electrical potential of the second conductive plate forces the current induced by the first conductive material plate to penetrate the surface of the tissue in contact with the conductive plates and prevents the induced current from flowing over the surface of the tissue.

14. The method of Claim 13, where before step A) a substance is placed on the tissue and in step B) the electrode is placed on the substance prepared; wherein the electric field of step B) has sufficient strength and duration to cause the tissue cell walls to become temporarily permeable allowing the substance to pass through them without damaging the tissue cells.

15. The method of Claim 13, wherein the activation signal applied in step B) has a frequency between 0.1 Hz and 50 MHz and a voltage variation between 1 V and 24 V.

16. The method of Claim 13, wherein the activation signal applied in step B) has a frequency between 0.1 Hz and 500 kHz and a voltage variation between 1 V and 24 V.

17. The method of Claim 13, wherein the electric field of step B) has an intensity between 2V/cm and 5V/cm.

18. The method of Claim 14, wherein the substance is ionically charged, and where the electric field is of equal or greater charge than the charge of the substance.

19. The method of Claim 14, where the substance is injected into the tissue.

20. The method of Claim 13, where more than one electrode is placed on different parts of a body.

21. A method of administering a substance with a tissue electrical stimulation electrode into a tissue, the method comprises the following steps:
a) placing the substance on the tissue;
b) locating the electrode of tissue electrical stimulation that includes:
- a first conductive plate; and
- a second conductive plate surrounding the first conductive plate; and where the first conductive plate and the second conductive plate are separated from each other, on the surface of the tissue with the conductive plates in contact with the tissue;
c) applying an activation signal to the first conductive plate of the electrode to generate an electric field that induces a current in the tissue; and
d) applying an electrical potential to the second electrode conductive plate to obtain an electrical potential differential with respect to the first conductive plate;
wherein the electric field of step c) is strong enough and durable enough to cause the tissue cell walls to become temporarily permeable to allow the substance to enter them without damaging the tissue cells.

22. The method of Claim 21, wherein the activation signal applied in step c) has a frequency between 0.1 Hz and 50 MHz and a voltage variation between 1 V and 24 V.

23. The method of Claim 21, wherein the activation signal applied in step c) has a frequency between 0.1 Hz and 500 kHz and a voltage variation between 1 V and 24 V.

24. The method of Claim 21, wherein the electric field of step c) has an intensity between 2V/cm and 5V/cm.

25. The method of Claim 21, where the substance is ionically charged, and where the electric field is of equal or greater charge than the charge of the substance.

26. The method of Claim 21, where the substance is injected into the tissue.

27. The method of Claim 21, where more than one electrode is placed on different parts of a body.

## Amended claims

### Amended claims under Art. 19.1 PCT

1. A tissue electrical stimulation electrode comprising:
- a first conductive plate; and
- a second conductive plate surrounding the first conductive plate, the second conductive plate establishes an electric potential barrier with the first conductive plate;
where the first conductive plate and the second conductive plate are separated from each other, the electric potential barrier corresponds to a region of the tissue with an electric field intensity formed by the electric potential applied by the second conductive plate, which opposes the passage of electrically charged particles, in a direction that depends on the sign of the electric charge with respect to the first conductive plate.

2. The electrode of Claim 1, wherein at least one of the conductive plates is made of sponge.

3. The electrode of Claim 1, wherein there is at least one additional conductive plate to the second conductive plate.

4. The electrode of Claim 1, wherein between the first conductive plate and the second conductive plate there is a sponge.

5. The electrode of Claim 4, wherein the sponge contains an ionically charged substance.

6. The electrode of Claim 1, wherein the first and second conductive plate consist of a conductive layer with a sponge layer.

7. The electrode of Claim 1, wherein the electrode body consists of a layer of insulating material supporting the first conductive plate and the second conductive plate.

8. The electrode of Claim 1, wherein the first conductive plate and the second conductive plate are coated with an insulating material.

9. The electrode of Claim 1, wherein around the second conductive plate there is at least one additional conductive plate, which surrounds the second conductive plate, in a concentric manner.

10. The electrode of Claim 1, for use in a tissue electrical stimulation machine.

11. The electrode of Claim 1, where at least one of the conductive plates is made of a sponge containing an ionically charged substance.

12. The electrode of Claim 11, where the ionically charged substance is a medicine.

13. A method of inducing an electric current with a tissue electrical stimulation electrode in a tissue, the method comprising the following steps:
A) locating the electrode of tissue electrical stimulation including:
- a first conductive plate; and
- a second conductive plate surrounding the first conductive plate which establishes an electric potential barrier with the first conductive plate; wherein the first conductive plate and the second conductive plate are separated from each other, on the tissue and with the conductive plates in contact with the tissue;
B) applying an activation signal to the first conductive plate of the electrode to generate an electric field inducing a current in the tissue; and
C) applying an electrical potential to the second electrode conductive plate to obtain an electrical potential differential with respect to the first conductive plate;
wherein the electrical potential of the second conductive plate forces the current induced by the first conductive material plate to penetrate the surface of the tissue in contact with the conductive plates and prevents the induced current from flowing over the surface of the tissue.

14. The method of Claim 13, wherein before step A) a substance is placed on the tissue and in step B) the electrode is placed on the substance prepared; wherein the electric field of step B) has sufficient strength and duration to cause the tissue cell walls to become temporarily permeable to allow the substance to pass through them without damaging the tissue cells.

15. The method of Claim 13, wherein the activation signal applied in step B) has a frequency between 0.1 Hz and 50 MHz and a voltage variation between 1 V and 24 V.

16. The method of Claim 13, wherein the activation signal applied in step B) has a frequency between 0.1 Hz and 500 kHz and a voltage variation between 1 V and 24 V.

17. The method of Claim 13, wherein the electric field of step B) has an intensity between 2V/cm and 5V/cm.

18. The method of Claim 14, where the substance is ionically charged, and where the electric field is of equal or greater charge than the charge of the substance.

19. The method of Claim 14, where the substance is injected into the tissue.

20. The method of Claim 13, where more than one electrode is placed on different parts of a body.

21. A method of administering an ionically charged substance with a tissue electrical stimulation electrode into a tissue, the method comprising the following steps:
a) placing the ionically charged substance on the tissue;
b) locating the electrode of tissue electrical stimulation including:
- a first conductive plate; and
- a second conductive plate surrounding the first conductive plate which establishes an electric potential barrier with the first conductive plate; and where the first conductive plate and the second conductive plate are separated from each other, on the surface of the tissue with the conductive plates in contact with the tissue;
c) applying an activation signal to the first conductive plate of the electrode to generate an electric field inducing a current in the tissue; and
d) applying an electric potential to the second electrode conductive plate to obtain an electric potential differential with respect to the first conductive plate;
wherein the electric field of step c) is strong enough and durable enough to cause the tissue cell walls to become temporarily permeable allowing the ionically charged substance to enter them without damaging the tissue cells.

22. The method of Claim 21, wherein the activation signal applied in step c) has a frequency between 0.1 Hz and 50 MHz and a voltage variation between 1 V and 24 V.

23. The method of Claim 21, wherein the activation signal applied in step c) has a frequency between 0.1 Hz and 500 kHz and a voltage variation between 1 V and 24 V.

24. The method of Claim 21, wherein the electric field of step c) has an intensity between 2V/cm and 5V/cm.

25. The method of Claim 21, wherein the electric field is equal to or greater than the charge of the ionically charged substance.

26. The method of Claim 21, where the ionically charged substance is injected into the tissue.

27. The method of Claim 21, where more than one electrode is placed on different parts of a body.

28. The method of Claim 21, where at least one of the conductive plates is made of a sponge containing an ionically charged substance.

29. The electrode of Claim 21, wherein between the first conductive plate and the second conductive plate there is a sponge.

30. The electrode of Claim 29, where the sponge contains an ionically charged substance.
